# EUROPÄISCHE PATENTSCHRIFT

(11) **EP 1 170 282 B1**
(45) Veröffentlichungstag und Bekanntmachung des Hinweises auf die Patenterteilung: **01.10.2003**
(21) Anmeldenummer: 01114779.0
(22) Anmeldetag: 26.06.2001
(51) Int. Cl.: C07C 253/00, C07C 255/05

(54) **Verfahren zur Herstellung von 1,3,6-Hexantricarbonitril**
Process for the preparation of hexane 1,3,6-tricarbonitrile
Procédé pour la préparation d'hexane 1,3,6-tricarbonitrile

(30) Priorität: 06.07.2000 DE 10032881
(43) Veröffentlichungstag der Anmeldung: 09.01.2002
(73) Patentinhaber: Bayer Aktiengesellschaft, 51368 Leverkusen (DE)
(72) Erfinder: Schelhaas, Michael, Dr., 50733 Köln (DE); Jautelat, Manfred, Dr., 51399 Burscheid (DE)

(56) Entgegenhaltungen:
- US-A- 2 307 643
- US-A- 3 375 237
- ROBERT M. COATES ET AL.: "Synthesis and Claisen rearrangement of alkoxyalkyl enol ethers. Evidence for a dipolar transition state" JOURNAL OF THE AMERICAN CHEMICAL SOCIETY., Bd. 109, Nr. 4, 18. Februar 1987 (1987-02-18), Seiten 1160-1170, XP002173970 DC US
- CHEMICAL ABSTRACTS, vol. 101, no. 21, 19. November 1984 (1984-11-19) Columbus, Ohio, US; abstract no. 191180v, RODRIGUEZ-HAHN, LYDIA ET AL.: "A study of the Thorpe-Ziegler reaction in very mild conditions." Seite 702; Spalte 1; XP002173971 & SYNTHETIC COMMUNICATIONS., Bd. 14, Nr. 10, 1984, Seiten 967-972, MARCEL DEKKER, INC., BASEL., CH ISSN: 0039-7911
- CHEMICAL ABSTRACTS, vol. 76, no. 15, 10. April 1972 (1972-04-10) Columbus, Ohio, US; abstract no. 85349n, USOVA, E. P. ET AL.: "Identification of high-boiling impurity in adipodinitrile, obtained from acrylonitrile." Seite 381; XP002173972 & ZH. PRIKL. KHIM., Bd. 44, Nr. 11, 1971, Seiten 2598-2599,
- DATABASE WPI Section Ch, Week 198801 Derwent Publications Ltd., London, GB; Class A60, AN 1988-004871 XP002173973 & JP 62 270550 A (ASAHI CHEM IND CO LTD), 24. November 1987 (1987-11-24)
- DATABASE WPI Section Ch, Week 197902 Derwent Publications Ltd., London, GB; Class A60, AN 1979-02876B XP002173974 & JP 53 135926 A (ASAHI CHEM IND CO LTD), 28. November 1978 (1978-11-28)

## Beschreibung

Die vorliegende Erfindung betrifft ein Verfahren zur Herstellung von Tricarbonitrilen, insbesondere 1,3,6-Hexantricarbonitril, in einer zweistufigen Synthese, wobei in der ersten Stufe über die Reaktion eines aliphatischen α-ω-Dinitrils in Gegenwart einer starken Base ein Intermediat erhalten wird, welches in einer zweiten Stufe mit Acrylnitril unter schwach basischen Bedingungen umgesetzt wird.

1,3,6-Hexantricarbonitril stellt ein wichtiges Vorprodukt für eine Reihe von industriell genutzter Produkte dar. Beispielsweise können durch Hydrolyse Tricarbonsäuren erhalten werden, die als Detergenzien Verwendung finden (DE-A-196 37 428). Die entsprechende Hydrogenierung des Trinitrils führt zu 1,3,6-Triaminohexan, welches dann in einem weiteren Schritt durch Phosgenierung zu 1,3,6-Triisocyanatohexan umgesetzt werden kann. Diese Verbindung dient als wichtiger Grundbaustein für die Polyurethan (PU)-Chemie wie z.B. zur Herstellung von PU-Klebstoffen oder PU-Lacken.

Die Verbindung 1,3,6-Hexantricarbonitril entsteht als Nebenprodukt bei der elektrochemischen Adiponitrilproduktion (JP-A-62270550). Das unerwünschte Nebenprodukt muss in einem aufwendigen Verfahren aus dem Destillationsrückstand isoliert werden. Dies ist zur Zeit die einzig mögliche industrielle Gewinnung von 1,3,6-Hexantricarbonitril.

In SU-A-194 088 ist die Nachstellung der elektrochemischen Adiponitrilsynthese beschrieben, wobei als Ausgangsprodukt ein ungesättigtes Intermediat des Adiponitrils zur Herstellung von 1,3,6-Hexantricarbonitril verwendet wird. Dieses Intermediat ist jedoch nicht industriell erhältlich.

Die Cyclisierung von Adiponitril zu 2-Amino-1-cyclopenten-1-carbonitril ist bekannt (Journal of the Chemical Society 1909, 700). Die Reaktion wird unter stark basischen Reaktionsbedingungen durchgeführt, dass heißt es werden Basen wie Alkalihydride, -amide oder Alkali-t-butylate eingesetzt.

Ebenfalls bekannt ist die Herstellung von 1,3,6-Hexantricarbonitril aus 2-Amino-1-cyclopenten-1-carbonitril und Acrylnitril in Gegenwart von elementarem Natrium (Journal of Applied Chemistry of the USSR, 1972, 2683-2684). Die Verwendung von elementarem Natrium und das damit verbundene erhöhte Sicherheitsrisiko schließt eine Übertragung auf einen großtechnischen Verfahrensprozess aus.

Es hat sich gezeigt, dass eine direkte Reaktion von Adiponitril mit Acrylnitril zu einer wenig selektiven Reaktion führt, da die deprotonierten Intermediate zu Dimerisierungen und Polymerisierungen führen können (Tsuruda, T.; O'Driscoll, K. F.; Eds. Structure and Mechanism in Vinyl Polymerization; Marcel Dekker: New York, 1969; Chapter 11, p. 345 ff.).

Die Aufgabe der vorliegenden Erfindung war somit die Bereitstellung eines Verfahrens zur selektiven Synthese von Tricarbonitrilen, insbesondere 1,3,6-Hexantricarbonitril, ausgehend von gängigen und leicht verfügbaren Ausgangsprodukten, sowie kontrollierbaren Reaktionsbedingungen.

Es wurde nun gefunden, dass eine zweistufige Reaktionsführung dieses Problem umgeht, indem zunächst in einem ersten Reaktionsschritt über ein aliphatisches α-ω-Dinitril unter stark basischen Bedingungen ein Intermediat erhalten wird, welches sich dann in einer zweiten Stufe selektiv und unter Ringöffnung mit Acrylnitril cyanoethylieren lässt. Unerwünschte Nebenreaktionen des Acrylnitrils werden nicht beobachtet.

Gegenstand der Erfindung ist somit ein Verfahren gemäß Anspruch 1 :

Verfahren zur Herstellung von Tricarbonitrilen der Formel (I), 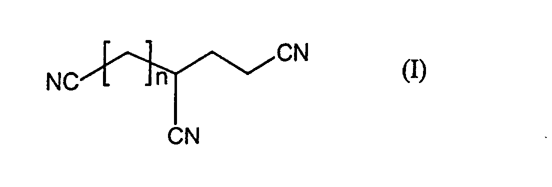 in welcher n für eine Zahl von 2 bis 11 steht,
insbesondere 1,3,6-Hexantricarbonitril (n = 3), gekennzeichnet dadurch, dass in einer ersten Stufe aus einem aliphatischen α-ω-Dinitril der Formel (II), 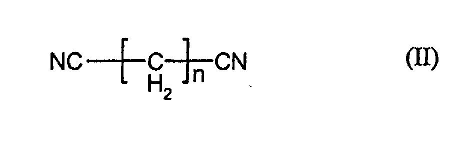 in welcher n für eine Zahl von 3 bis 12 steht,
in Gegenwart einer Base-, die
A) ein Alkalimetall, -hydrid,- amid, -alkoholat oder
B) ein Metalloxid, -hydroxid der 1. oder 2. Hauptgruppe unter Zusatz komplexbildender Lösemittel oder Phasentransferkatalysatoren
is, ein Intermediat der Formel (III) gebildet wird, 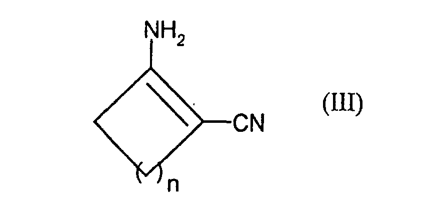 in welcher n für eine Zahl von 1 bis 10 steht,
welches anschließend in einem zweiten Schritt
a) unmittelbar ohne Aufarbeitung oder
b) isoliert, und anschließend unter Zusatz einer Base, die Kaliumcarbonat Natriumcarbonat, Natriumphosphat, Natriumhydroxid oder Kaliumhydroxid ist, ggf. unter Zugabe von Phasentransfer-Katalysatoren: Kronenethern oder Kryptanden,
mit Acrylnitril zum Trinitril der Formel (I) umgesetzt wird. 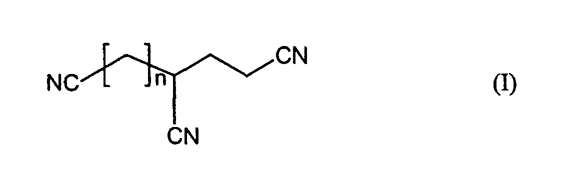 in welcher n für eine Zahl von 2 bis 11 steht,
insbesondere 1,3,6-Hexantricarbonitril (n = 3), dadurch gekennzeichnet, dass in einer ersten Stufe aus einem aliphatischen α-ω-Dinitril der Formel (II), 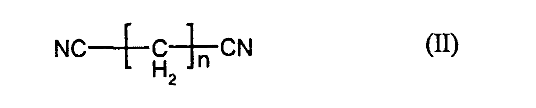 in welcher n für eine Zahl von 3 bis 12 steht,
in Gegenwart einer starken Base ein Intermediat gebildet wird, welches anschließend in einem zweiten Schritt durch Zugabe von Acrylnitril zum Trinitril der Formel (I) umgesetzt wird.

In einer bevorzugten Ausführungsform des erfindungsgemäßen Verfahrens wird in der ersten Stufe aus Adiponitril unter stark basischen Reaktionsbedingungen ein Intermediat erhalten, welches dann in einer zweiten Stufe mit Acrylnitril zu 1,3,6-Hexantricarbonitril reagiert.

Die Synthese wird bevorzugt als Eintopfreaktion durchgeführt.

Gegebenenfalls kann in dem erfmdungsgemäßen Verfahren das Intermediat isoliert werden und anschließend mit Acrylnitril zum Trinitril der Formel (I) umgesetzt werden.

Das Intermediat kann beispielsweise, wie die Auswertung der analytischen Daten gezeigt hat, als 2-Amino-1-cyclopenten-1-carbonitril der Formel (III), 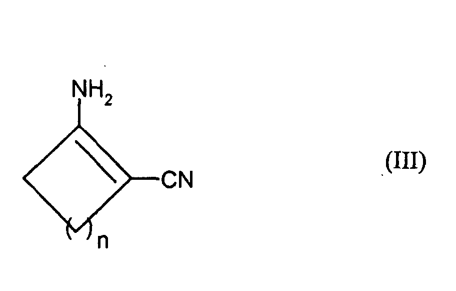 in welcher n für eine Zahl von 1 bis 10 steht,
identifiziert werden.

Die erste Stufe des erfindungsgemäßen Verfahrens wird in einem Temperaturbereich von 70 bis 120°C, bevorzugt von 80 bis 100°C, durchgeführt.

Geeignete starke Basen sind beispielsweise Alkalimetalle, -hydride, -amide oder -alkoholate wie Alkali-tert-butylate, bevorzugt ist Kalium-tert-butylat. Über eine ausreichende Basizität verfügen auch Metalloxide und -hydroxide der 1. und 2. Hauptgruppe des Periodensystems in komplexbildenden Lösungsmitteln wie Polyethylenglycolen, bevorzugt sind Diglyme, Ethylenglycoldimethylether oder Polyethylenglycoldimethylether M 500® (Aldrich), oder phasentransferkatalysierte Systeme, bevorzugt thermostabile Phasentransferkatalysatoren, besonders bevorzugt Aliquat 175® bzw. Aliquat 336® (Cognis) oder Aliplex 186BD® (Cognis).

Die im erfindungsgemäßen Verfahren verwendete starke Base wird in einer Menge von 0,5 bis 2, bevorzugt von 1 bis 1,5 Equivalenten, bezogen auf das α-ω-Dinitril, eingesetzt.

Nach beendeter Umsetzung des α-ω-Dinitrils, unter Bildung eines Intermediates, wird bei einer geringeren Basizität des Reaktionsmediums, verglichen mit der ersten Umsetzung, Acrylnitril zugegeben. Bevorzugt wird vor der Zugabe von Acrylnitril eine equimolare Menge Wasser zugesetzt.

Die zweite Stufe der Synthese verläuft in einem Temperaturbereich von 0 bis 120°C, bevorzugt von 10 bis 70°C.

Wird gegebenenfalls das Intermediat der Formel (III) isoliert, kann dieses in einer separaten zweiten Stufe mit Acrylnitril und unter Zugabe einer schwachen Base zu 1,3,6-Hexantricarbonitril der Formel (I) cyanoethyliert werden, wobei 1 bis 1,5 Equivalente Acrylnitril, bezogen auf die molare Menge an isoliertem Intermediat der Formel (III), der Reaktion zugegeben werden.

Geeignete schwache Basen sind beispielsweise Kaliumcarbonat, Natriumcarbonat, Natriumphosphat, Natriumhydroxid oder Kaliumhydroxid oder mit Phasentransfer-Katalysatoren, wie quartäre Ammonium-, Phosphonium- u. a. Onium-Verbindungen oder Kronenether und Kryptanden, geführte Systeme. Bevorzugt wird als Phasentransferkatalysator ein quartäres Ammoniumsalz (Aliquat 336) in wäßriger Natronlauge verwendet.

Als Reaktionsmedium für das erfindungsgemäße Verfahren geeignet sind beispielsweise inerte organische Lösungsmittel wie Benzol oder Petrolether, bevorzugt wird als Lösungsmittel Toluol verwendet.

Das erfindungsgemäße Verfahren kann sowohl inert als auch in der Gegenwart von Sauerstoff und bei einem Druck von 1 bis 50 bar, vorzugsweise bei Atmosphärendruck, durchgeführt werden. Die Behandlung des Dinitrils mit Basen wird vorteilhafterweise unter Inertbedingungen durchgeführt.

Die weitere Aufarbeitung des erfindungsgemäß hergestellten Tricarbonitrils erfolgt nach dem Fachmann bekannten Standardmethoden.

### Beispiele

Die angegebenen Selektivitäten beschreiben das Verhältnis von Produkt zu Umsatz. In den Beispielen 1 bis 4 kann nicht umgesetztes Intermediat in einem weiteren Schritt aufgearbeitet und erneut der Reaktion zugeführt werden.

### Beispiel 1 Eintopfsynthese zur Herstellung von 1,3,6-Hexantricarbonitril

Eine Mischung aus 2,16 g (20 mmol) Adiponitril, 1,8 g (32 mmol) KOH-Pulver, 200 mg (0,59 mmol) Tetrabutylammoniumhydrogensulfat in 50 ml Toluol wird unter Argon über eine Reaktionszeit von 2 h auf 100°C erhitzt. Nach dem Abkühlen auf Raumtemperatur (RT) werden 1,17 g (22 mmol) Acrylnitril in 10 ml Toluol zugegeben und die Mischung wird bei RT für 2 Stunden gerührt. Nach Zugabe von Wasser wird die organische Phase abgetrennt, getrocknet, das Lösungsmittel abdestilliert und der Rückstand gaschromatographisch untersucht.
Ausbeute an Trimeren: 17 %
Selektivität bezüglich Trimeren: 40 %

### Beispiel 2 Eintopfsynthese zur Herstellung von 1,3,6-Hexantricarbonitril

Unter Argon werden 5,41 g (50 mmol) Adiponitril bei 65°C zu einer Suspension aus 5,6 g (50 mmol) Kalium-*tert*.-butylat in 50 ml Toluol gegeben und die Mischung für eine Stunde unter Rückfluss erhitzt. Zu der auf Raumtemperatur abgekühlten Mischung werden langsam 4 ml (60 mmol) Acrylnitril gegeben, die Mischung wird 1 Stunde bei RT nachgerührt und dann mit Wasser versetzt. Die Phasen werden getrennt, die wässrige Phase mit Essigsäureethylester extrahiert, die vereinigten organischen Phasen getrocknet, das Lösungsmittel abdestilliert und der Rückstand gaschromatographisch untersucht.
Ausbeute an Trimeren: 9,3 %
Selektivität bezüglich Trimeren: 18 %

### Beispiel 3 Eintopfsynthese zur Herstellung von 1,3,6-Hexantricarbonitril

Unter Argon werden 5,41 g (50 mmol) Adiponitril bei 65°C zu einer Suspension aus 5,6 g (50 mmol) Kalium-*tert*.-butylat in 50 ml Toluol gegeben und die Mischung für eine Stunde unter Rückfluss erhitzt. Zu der auf Raumtemperatur abgekühlten Mischung werden 0,9 ml (50 mmol) Wasser und danach langsam 4 ml (60 mmol) Acrylnitril gegeben, die Mischung wird 1 Stunde bei RT nachgerührt und dann mit Wasser versetzt. Die Phasen werden getrennt, die wässrige Phase mit Essigsäureethylester extrahiert, die vereinigten organischen Phasen getrocknet, das Lösungsmittel abdestilliert und der Rückstand gaschromatographisch untersucht.
Ausbeute an Trimeren: 35 %
Selektivität bezüglich Trimeren: 47 %

### Beispiel 4 Eintopfsynthese zur Herstellung von 1,3,6-Hexantricarbonitril

Unter Argon werden 5,41 g (50 mmol) Adiponitril bei 65°C zu einer Suspension aus 5,6 g (50 mmol) Kalium-*tert*.-butylat in 50 ml Toluol gegeben und die Mischung für eine Stunde unter Rückfluss erhitzt. Zu der auf Raumtemperatur abgekühlten Mischung werden 0,9 ml (50 mmol) Wasser und danach langsam 4 ml (60 mmol) Acrylnitril, gelöst in 20 ml Toluol, gegeben, die Mischung wird 1 Stunde bei RT nachgerührt und dann mit Wasser versetzt. Die Phasen werden getrennt, die wässrige Phase mit Essigsäureethylester extrahiert, die vereinigten organischen Phasen getrocknet, das Lösungsmittel abdestilliert und der Rückstand gaschromatographisch untersucht.
Ausbeute an Trimeren: 43 %
Selektivität bezüglich Trimeren: 69 %

### Beispiel 5 Herstellung von 1,3,6-Hexantricarbonitril: Reaktion über die Isolierung von 2-Amino-1-cyclopenten-1-carbonitril

Es werden 216 mg (2 mmol) Adiponitril und 128 mg (3,2 mmol) Natriumhydroxid Micropills in 5 ml Polyethylenglycoldimethylether M 500 (Aldrich) (als Löslichkeitsvermittler) für 22 Stunden auf 95°C erhitzt. Die Mischung wird mit 3 ml Wasser versetzt und mit Toluol extrahiert.
Ausbeute an 2-Amino-1-cyclopenten-1-carbonitril: 84 %

Analytik durch NMR-Spektroskopie:
¹H-NMR (400MHz, d⁶-DMSO):
δ (ppm): 1,7-1,8 (m, 2H); 2,3-2,4 (m, 4H); 6,4 (s, br, 2H).
¹³C-NMR (100MHz, d⁶-DMSO):
δ (ppm): 22,0; 31,2; 34,2; 68,3; 120,3; 164,4.
Zu einer Mischung aus 1,08 g (10 mmol) 2-Amino-1-cyclopenten-1-carbonitril, 2 ml 45%iger Natronlauge, 100 mg Aliquat 336 (Cognis) und 6 ml Toluol wird bei RT 583 mg (11 mmol) Acrylnitril gegeben und bei RT 22 h gerührt. Die Mischung wird mit Wasser verdünnt, die Phasen werden getrennt, die organische Phase getrocknet, das Lösungsmittel abdestilliert und der Rückstand gaschromatographisch untersucht.
Ausbeute an Trimeren: 63,2 %
Selektivität bezüglich Trimeren: 75 %

### Beispiel 6: Herstellung von 1,3,6-Hexantricarbonitril über die Isolierung von 2-Amino-1-cyclopenten-1-carbonitril

Isolierung von 2-Amino-1-cyclopenten-1-carbonitril wie unter Beispiel 5:
Zu einer Mischung aus 1,08 g (10 mmol) 2-Amino-1-cyclopenten-1-carbonitril (aus Beispiel 5), 2 ml (33,7 mmol) 45 %iger Natronlauge, 100 mg Aliquat 336 und 6 ml Toluol wird bei RT 583 mg (11 mmol) Acrylnitril gegeben und bei 50°C 22 h gerührt. Die Mischung wird mit Wasser verdünnt, die Phasen werden getrennt, die organische Phase getrocknet, das Lösungsmittel abdestilliert und der Rückstand gaschromatographisch untersucht.
Ausbeute an Trimeren: 66,9 %
Selektivität bezüglich Trimeren: 79 %

### Beispiel 7: Herstellung von 1,3,6 1,3,6-Hexantricarbonitril über die Isolierung von 2-Amino-1-cyclopenten-1-carbonitril

Isolierung von 2-Amino-1-cyclopenten-1-carbonitril wie unter Beispiel 5:
Eine Mischung aus 32 mg (0,3 mmol) 2-Arnino-l-cyclopenten-1-carbonitril (aus Beispiel 5), 3 ml (48 mmol) 45 %ige Natronlauge, 0,5 mg (0,0015 mmol) Tetrabutylammoniumhydrogensulfat und 16 mg (0,3 mmol) Acrylnitril in 7 ml Toluol werden für 2 h auf 50°C erhitzt. Nach dem Abkühlen wird die Toluolphase gaschromatographisch untersucht.
Ausbeute an Trimeren: 85 %
Selektivität bezüglich Trimeren: 90 %

## Patentansprüche

1. Verfahren zur Herstellung von Tricarbonitrilen der Formel (I), 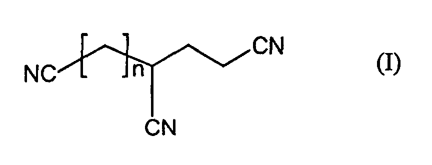 in welcher n für eine Zahl von 2 bis 11 steht,
insbesondere 1,3,6-Hexantricarbonitril (n = 3), **gekennzeichnet dadurch, dass** in einer ersten Stufe aus einem aliphatischen α-ω-Dinitril der Formel (II), 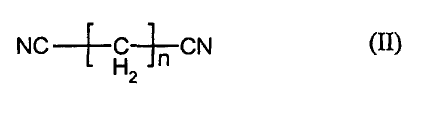 in welcher n für eine Zahl von 3 bis 12 steht,
in Gegenwart einer Base-, die
A) ein Alkalimetall, -hydrid,- amid, -alkoholat oder
B) ein Metalloxid, -hydroxid der 1. oder 2. Hauptgruppe unter Zusatz komplexbildender Lösemittel oder Phasentransferkatalysatoren
ist, ein Intermediat der Formel (III) gebildet wird, 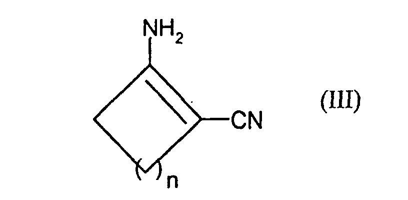 in welcher n für eine Zahl von 1 bis 10 steht,
welches anschließend in einem zweiten Schritt
a) unmittelbar ohne Aufarbeitung oder
b) isoliert, und anschließend unter Zusatz einer Base, die Kaliumcarbonat, Natriumcarbonat, Natriumphosphat, Natriumhydroxid oder Kaliumhydroxid ist, ggf. unter Zugabe von Phasentransfer-Katalvsatoren, Kronenethern oder Kryptanden_{L}
mit Acrylnitril zum Trinitril der Formel (I) umgesetzt wird.

2. Verfahren nach Anspruch 1, **dadurch gekennzeichnet, dass** in der ersten Stufe als aliphatisches α-ω-Dinitril Adiponitril eingesetzt wird.

3. Verfahren nach Anspruch 1, **dadurch gekennzeichnet, dass** die Synthese ohne Isolierung des Intermediats der Formel (III) als Eintopfreaktion a) durchgeführt wird.

4. Verfahren nach Anspruch 1, **dadurch gekennzeichnet, dass** die starke Base im ersten Schritt zur Bildung des Intermediats der Formel (III) in einer Menge von 0,5 bis 2, bevorzugt von 1 bis 1,5 Equivalenten, bezogen auf das α-ω-Dinitril, eingesetzt wird.

5. Verfahren nach Anspruch 1, **dadurch gekennzeichnet, dass** die erste Reaktionsstufe in einem Temperaturbereich von 70 bis 120°C durchgeführt wird.

6. Verfahren nach Anspruch 1, **dadurch gekennzeichnet, dass** der zweite Reaktionsschritt in einem Temperaturbereich von 0 bis 120°C durchgeführt wird.

## Claims

1. Process for the preparation of tricarbonitriles of the formula (I) 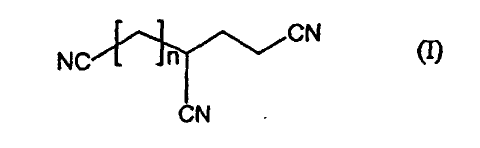 in which n represents a number from 2 to 11,
in particular 1,3,6-hexanetricarbonitrile (n = 3), **characterized in that** in a first stage, from an aliphatic α-ω-dinitrile of the formula (II) 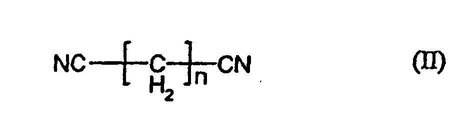 in which n represents a number from 3 to 12,
in the presence of a base which is
A) an alkali metal hydride, amide, alcoholate or
B) a metal oxide, hydroxide of the 1et or 2nd main group, with the addition of complexing solvents or phase transfer catalysts,
an intermediate of the formula (III) is formed 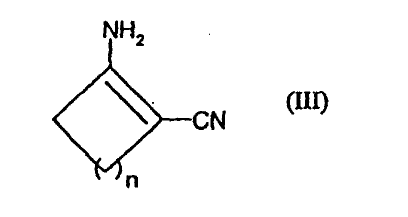 in which n represents a number from 1 to 10,
which is then reacted in a second step
a) directly without working up or
b) is isolated, and subsequently, with the addition of a base which is potassium carbonate, sodium carbonate, sodium phosphate, sodium hydroxide or potassium hydroxide, optionally with the addition of phase transfer catalysts, crown ethers or cryptands,
with acrylonitrile to give the trinitrile of the formula (I).

2. Process according to claim 1, **characterized in that** adiponitrile is employed as the aliphatic α-ω-dinitrile in the first stage.

3. Process according to claim 1, **characterized in that** the synthesis is carried out as a one-pot reaction a) without isolation of the intermediate of the formula (III).

4. Process according to claim 1, **characterized in that** the strong base in the first step for formation of the intermediate of the formula (III) is employed in an amount of 0.5 to 2, preferably 1 to 1.5 equivalents, based on the α-ω-dinitrile.

5. Process according to claim 1, **characterized in that** the first reaction stage is carried out in a temperature range of 70 to 120°C.

6. Process according to claim 1, **characterized in that** the second reaction step is carried out in a temperature range of 0 to 120°C.

## Revendications

1. Procédé de préparation de tricarbonitriles de la formule (I) : 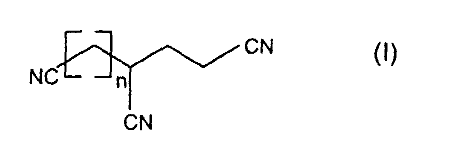 dans laquelle n représente un nombre allant de 2 à 11,
en particulier du 1,3,6-hexanetricarbonitrile (n = 3), **caractérisé en ce que** dans une première étape, on forme un intermédiaire de la formule (III) : 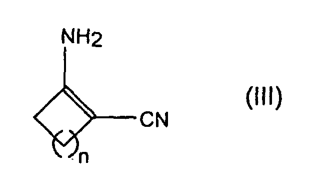 dans laquelle n représente un nombre allant de 1 à 10,
à partir d'un α,ω-dinitrile aliphatique de la formule (II) : 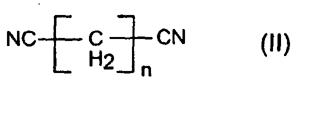 dans laquelle n représente un nombre allant de 3 à 12,
en présence d'une base, qui est
A) un métal, hydrure, amidure, alcoolate alcalin, ou
B) un oxyde, hydroxyde métallique des groupes principaux 1 ou 2 avec addition d'un solvant complexant ou de catalyseurs de transfert de phase,
lequel intermédiaire est ensuite dans une deuxième étape,
a) directement sans traitement, ou
b) isolé, et ensuite avec addition d'une base, qui est le carbonate de potassium, le carbonate de sodium, le phosphate de sodium, l'hydroxyde de sodium ou l'hydroxyde de potassium, le cas échéant avec addition de catalyseurs de transfert de phase, d'éthers couronnes ou de cryptants, mis à réagir avec l'acrylonitrile en trinitrile de la formule (I).

2. Procédé suivant la revendication 1, **caractérisé en ce que** dans la première étape, on met en oeuvre comme α,ω-dinitrile aliphatique, l'adiponitrile.

3. Procédé suivant la revendication 1, **caractérisé en ce que** l'on réalise la synthèse sans isolement de l'intermédiaire de la formule (III) sous la forme d'une réaction one-pot a).

4. Procédé suivant la revendication 1, **caractérisé en ce que** la base forte est mise en oeuvre dans la première étape pour la formation de l'intermédiaire de la formule (III), en une quantité allant de 0,5 à 2, de préférence de 1 à 1,5 équivalent, sur base de l'α,ω-dinitrile.

5. Procédé suivant la revendication 1, **caractérisé en ce que** l'on réalise la première étape de la réaction dans un intervalle de température allant de 70 à 120°C.

6. Procédé suivant la revendication 1, **caractérisé en ce que** l'on réalise la deuxième étape de réaction dans un intervalle de température allant de 0 à 120°C.
